Europäisches Patentamt

European Patent Office

Office européen des brevets

(11) Numéro de publication: **0 290 361**
**A1**

## (12) DEMANDE DE BREVET EUROPEEN

(21) Numéro de dépôt: **88420149.2**

(51) Int. Cl.⁴: **C 02 F 3/30**

(22) Date de dépôt: **06.05.88**

(30) Priorité: **07.05.87 FR 8706880**

(43) Date de publication de la demande:
**09.11.88 Bulletin 88/45**

(84) Etats contractants désignés:
**AT BE CH DE ES GB GR IT LI LU NL SE**

(71) Demandeur: **Jullien, Antonin**
**1, rue Henri Gorjus**
**F-69004 Lyon (FR)**

**Morel, Pierre**
**57, avenue Sainte Marie**
**F-94160 Saint Mande (FR)**

**Jullien, Yasmine**
**1, rue Henri Gorjus**
**F-69004 Lyon (FR)**

**Chevalier épouse Jullien, Claudette**
**1, rue Henri Gorjus**
**F-69004 Lyon (FR)**

(72) Inventeur: **Jullien, Antonin**
**1, rue Henri Gorjus**
**F-69004 Lyon (FR)**

**Morel, Pierre**
**57, avenue Sainte Marie**
**F-94160 Saint Mande (FR)**

**Jullien, Yasmine**
**1, rue Henri Gorjus**
**F-69004 Lyon (FR)**

**Chevalier épouse Jullien, Claudette**
**1, rue Henri Gorjus**
**F-69004 Lyon (FR)**

(74) Mandataire: **Maureau, Philippe et al**
**Cabinet Germain & Maureau Le Britannia - Tour C 20, bld**
**Eugène Déruelle Boîte Postale 3011**
**F-69392 Lyon Cédex 03 (FR)**

(54) **Procédé de fermentation méthanique de matières organiques et dispositif pour sa mise en oeuvre.**

(57) Ce procédé consiste à réaliser successivement l'oxygénation des boues pendant un jour dans une cuve (A), une préfermentation des boues en conditions aérobie puis anaérobie pendant un jour dans une cuve (B), une fermentation en conditions anaérobie pendant vingt jours dans une cuve (C) et, éventuellement, une fermentation complète en conditions anaérobie pendant soixante jours, les boues subissant la première phase d'oxygénation étant formées par moitié par des boues préparées à partir des matières organiques à traiter et pour moitié par des boues recyclées après avoir subi la première phase de fermentation.

Application à la production de méthane à partir de déchets organiques.

FIG.2

EP 0 290 361 A1

## Description

## PROCEDE DE FERMENTATION METHANIQUE DE MATIERES ORGANIQUES ET DISPOSITIF POUR SA MISE EN OEUVRE

La présente invention a pour objet un procédé de fermentation méthanique de matières organiques et un dispositif pour sa mise en oeuvre.

Ce procédé vise plus spécialement à permettre la fermentation de matières organiques constituées par des déchets urbains, tels que des ordures ménagères, des déchets agricoles, forestiers, des algues d'eau douce ou des algues marines.

Suivant son stade de mise en oeuvre, ce procédé permet de réaliser une dégradation à peu près totale des matières organiques pour obtenir une production maximale de gaz ou une dégradation à un taux de l'ordre de 50 à 60 % permettant d'obtenir des boues de rejet de méthanisation destinées à former un engrais naturel.

Ce procédé consiste à réaliser successivement l'oxygénation des boues pendant un jour, une préfermentation des boues en conditions aérobie puis anaérobie pendant un jour, une fermentation en conditions anaérobie pendant vingt jours et, éventuellement, une fermentation complète en conditions anaérobie pendant soixante jours, les boues subissant la première phase d'oxygénation étant formées par moitié par des boues préparées à partir des matières organiques à traiter et pour moitié par des boues recyclées après avoir subi la première phase de fermentation.

Il est à noter qu'à l'issue de la première phase de fermentation, la dégradation des matières organiques est réalisée à une taux de 55 %, alors qu'à l'issue de la seconde phase de fermentation, la dégradation des matières organiques est presque totale puisqu'elle dépasse 97 à 98 %.

Selon un mode de mise en oeuvre, ce procédé consiste :
- au cours de la phase d'oxygénation, à réaliser une insufflation d'air dans la cuve dans laquelle se trouvent les boues, cette insufflation étant de 60 l/heure et par m³, et la teneur en matières sèches des boues étant de 180 g/litre,
- au cours de la phase de préfermentation à réaliser une agitation des boues par insufflation du gaz produit par la réaction et à maintenir la température à 37° C,
- au cours de la première phase de fermentation à agiter périodiquement les boues à l'aide du gaz produit par la réaction, cette phase se déroulant dans une enceinte isolée thermiquement, et
- au cours de la seconde phase de fermentation à réaliser une agitation périodique des boues à l'aide du gaz dégagé.

Conformément à une autre caractéristique de ce procédé, celui-ci consiste quotidiennement à prélever un volume de boues ayant subi la première phase de fermentation égal au volume de boues devant subir la phase d'oxygénation, à recycler la moitié de ces boues pour la phase d'oxygénation, et à diriger l'autre moitié de ces boues soit vers une installation de réalisation d'engrais, soit vers la seconde cuve de fermentation.

Un dispositif pour la mise en oeuvre de ce procédé comprend quatre cuves dont les trois premières sont décalées verticalement les unes par rapport aux autres, la première cuve qui correspond à la cuve la plus haute étant la cuve d'oxygénation, la seconde cuve communiquant avec celle-ci par un conduit équipé d'une vanne étant la cuve de préfermentation, la troisième cuve servant à la première fermentation étant reliée à la cuve précédente par un conduit sur lequel est montée une vanne, la quatrième cuve étant reliée à la troisième cuve par un conduit sur lequel est disposée une pompe, le volume de chacune des première et deuxième cuves étant égal au vingtième du volume de la troisième cuve, et le volume de la quatrième cuve étant inférieur au volume de la troisième cuve.

Avantageusement, sur le conduit reliant les troisième et quatrième cuves, est monté un deshydrateur destiné à augmenter la concentration des boues en matière sèche, et le volume de la quatrième cuve est égal à 0,583 fois le volume de la troisième cuve.

Selon une autre caractéristique de ce dispositif, le fond de chaque cuve est incliné de l'extrémité amont vers l'extrémité aval de celle-ci d'une valeur de l'ordre de 1 à 2 cm/m.

En outre, le fond de chacune des cuves est équipé de busettes alimentées par un compresseur, les busettes de la première cuve étant alimentées par de l'air prélevé à l'extrérieur de celle-ci, tandis que les busettes des autres cuves sont alimentées par du gaz contenu dans chacune de celles-ci.

Cette alimentation en air ou en gaz de chacune des cuves assure, d'une part, le brassage des boues, maintenant une bonne homogénéité de celles-ci sans formation de blocs solides et, d'autre part, le déplacement des boues le long du fond de chaque cuve de l'extrémité amont vers l'extrémité aval de celle-ci.

Il est également possible d'assurer le brassage des boues et le déplacement de celles-ci dans les cuves de fermentation à l'aide d'agitateurs mécaniques verticaux, ou de combiner les actions d'agitateurs mécaniques et de busettes, les agitateurs mécaniques étant par exemple disposés à l'extrémité amont d'un tronçon de la cuve considérée et les busettes étant disposées dans la seconde partie de ce même tronçon de cuve.

Afin d'assurer le maintien des conditions optimales de déroulement de la réaction dans chacune des cuves, la seconde cuve est équipée de moyens de chauffage et de régulation de la température à 37° C, tandis que la troisième cuve est équipée de moyens d'isolation thermique assurant un maintien en température des boues transférées depuis la seconde cuve.

Selon une autre caractéristique de l'invention, à l'extrémité aval de la troisième cuve, c'est-à-dire de la cuve correspondant à la première phase de fermentation est connecté un conduit de retour des boues vers la première cuve ou cuve d'oxygénation, ce retour des boues étant réalisé à l'aide d'une pompe.

De toute façon, l'invention sera bien comprise à l'aide de la description qui suit, en référence au dessin schématique annexé représentant, à titre d'exemple non limitatif, une forme d'exécution d'un dispositif pour la mise en oeuvre de ce procédé :

Figure 1 est une vue d'un schéma bloc montrant les différentes parties du dipositif ;

Figure 2 est une vue en coupe longitudinale par un plan vertical de l'installation ;

Figure 3 en est une vue en coupe longitudinale par un plan horizontal selon la ligne III-III de figure 2.

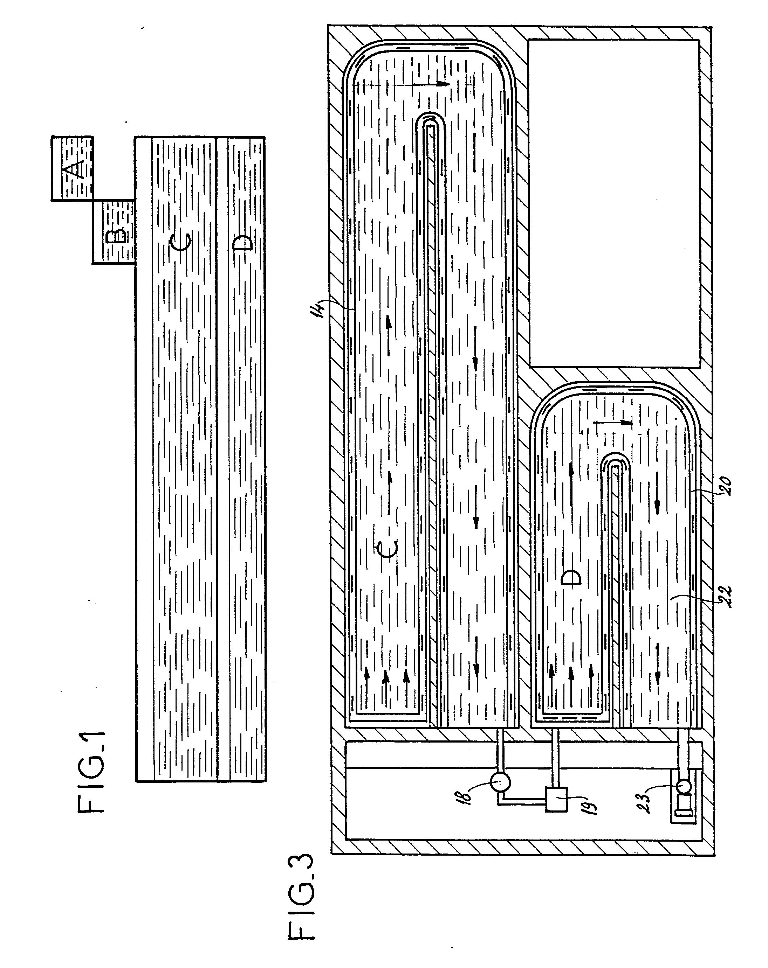

Comme montré à la figure 1, l'installation comprend essentiellement quatre cuves A, B, C et D, la cuve A étant la cuve d'oxygénation dans laquelle les boues demeurent un jour, la cuve B étant une cuve de préfermentation dans laquelle les boues demeurent pendant un jour, la cuve C étant une cuve de première fermentation dans laquelle les boues demeurent vingt jours et la cuve D étant une cuve de fermentation complète des matières organiques dans laquelle les boues demeurent soixante jours.

Compte tenu des temps de séjour respectifs des boues dans les différentes cuves, et compte tenu du fait qu'une partie des boues contenues dans la cuve C sont recyclées dans la cuve A, le volume des cuves A et B est égal à 1/20e de celui de la cuve C et le volume de la cuve D est égal à 0,583 fois celui de la cuve C.

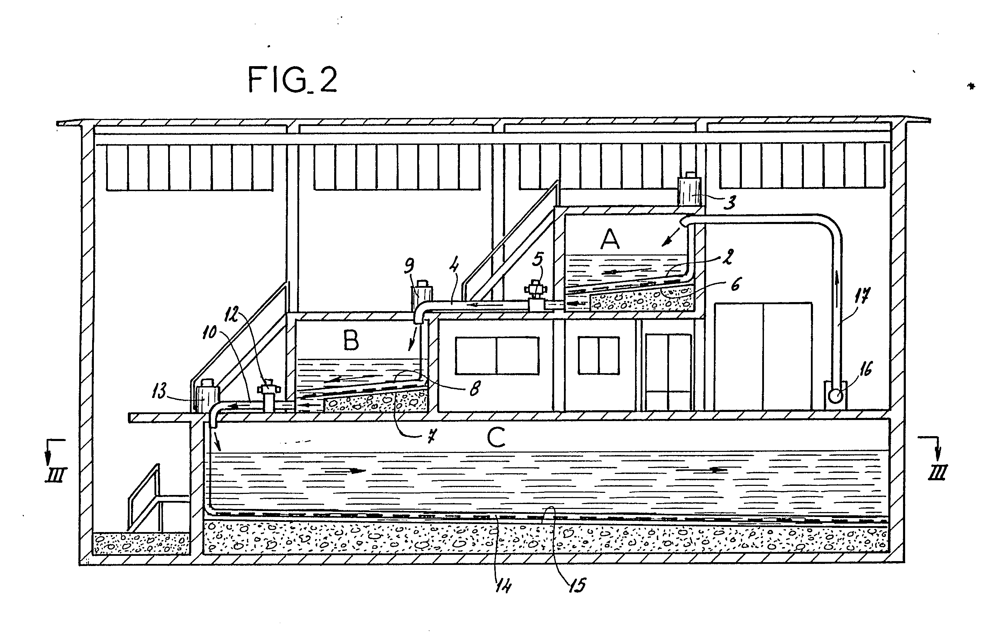

Comme cela est montré aux figures 2 et 3, les trois cuves A, B et C sont superposées, le passage de matières de l'une à l'autre se faisant par gravité, alors que la cuve D est disposée latéralement à la cuve C, le reste du bâtiment pouvant avantageusement être mis à profit pour recevoir les installations de préparation des boues.

L'alimentation journalière en boues de la cuve A se fait pour 50 % à l'aide de boues préparées à partir de matières organiques à traiter et pour 50 % à partir de boues ayant déjà été traitées dans la cuve C. La teneur en matières sèches est de l'ordre de 180 g/l. Au cours de leur séjour à l'intérieur de la cuve A, les boues sont oxygénées et brassées par insufflation d'air à l'aide d'une rampe 2 portant une pluralité de busettes alimentées à partir d'un compresseur 3 prenant l'air à l'extérieur de la cuve, et le distribuant à raison de 60 par heure et par m³. Cette cuve étant en conditions aérobie, la température des boues n'est pas contrôlée. Cependant, il convient de noter que ces boues sont déjà à une température supérieure à la température ambiante, puisque les boues recyclées de la cuve C sont à 37°C et que les boues provenant de la cuve de préparation ont subi un échauffement au cours de cette préparation.

Le pH des boues à l'intérieur de cette cuve est de l'ordre de 7,5 à 8. A l'expiration d'un temps de séjour de l'ordre de 24 heures, les boues sont transférées de la cuve A dans la cuve B par une conduite 4, sur laquelle est disposée une vanne électro-pneumatique 5, ce transfert étant facilité par le fait que le fond 6 de la cuve A est incliné de son extrémité amont vers son extrémité aval.

La cuve B possède la même structure générale que la cuve A, ainsi que le même volume, cette cuve étant équipée d'un fond incliné 7, d'un conduit 8 équipé de busettes distribuant du gaz repris par un compresseur 9 afin d'assurer l'agitation des boues pour maintenir les matières organiques en suspension.

Lors du transfert des boues de la cuve A dans la cuve B, cette dernière est en conditions aérobie. Cependant, au fur et à mesure que le gaz carbonique se forme, il chasse l'air de la cuve et la met en conditions anaérobie. Cette cuve est également équipée de moyens de régulation de la température à 37°, non représentés au dessin.

La formation de gaz carbonique entraîne le chute du pH qui, à l'expiration de la période de séjour de 24 heures est de l'ordre de 6,5. Au cours des dernières heures de cette phase de préfermentation, la composition du gaz contenu dans la cuve est de l'ordre de 18 % de $CH_4$ et de 82 % de $CO_2$.

La dégradation des matières organiques ayant commencé, il en résulte un léger abaissement, de l'ordre de 2 à 3 %, soit environ 5 g/l de la teneur en matières sèches. De ce fait, au moment de leur transfert dans la cuve C, les boues ont une teneur en matières sèches de 175 g/l. Le transfert des boues de la cuve B dans la cuve C est réalisé dans une conduite 10 sur laquelle est montée une vanne électro-pneumatique 12. Le temps de rétention dans cette cuve est de vingt jours.

A intervalle de temps déterminé, un compresseur 13 aspire le gaz produit dans la cuve pour l'insuffler dans un conduit 14 équipé de busettes, disposé contre le fond 15 de la cuve. Cette insufflation de gaz assure un brassage du .mélange maintenant les matières organiques en suspension et assure un transfert des boues de l'extrémité amont vers l'extrémité aval de la cuve, du fait de l'inclinaison du fond 15 de l'ordre de 1 cm/m.

A leur entrée dans la cuve, la teneur en matières sèches des boues est de l'ordre de 175 g/l avec 60 % de matières organiques soit 105 g/l et 40 % de matières minérales soit 70 g/l.

La dégradation des matières organiques dans la cuve C, est de l'ordre de 56 % si bien qu'au moment du rejet des boues, la teneur en matières sèches est de l'ordre de 116 g/l, à savoir 46 g/l pour les matières organiques et 70 g/l pour les matières minérales.

D'un point de vue de la structure de la cuve, il faut remarquer que, pour assurer le maintien des boues à une température de 37°C, son enceinte est parfaitement isolée thermiquement.

L'opération de méthanisation se déroulant dans cette cuve, ramène le pH de l'état acide à l'état basique à une valeur de 7,50, le gaz produit contenant environ 68 % de $CH_4$ et 32 % de $CO_2$.

Chaque jour, 1/20e du contenu de la cuve C est évacué de celle-ci. La moitié du volume évacué est recyclée dans la cuve A par l'intermédiaire d'une pompe 16 et d'une conduite 17. Pour l'autre moitié du volume évacué, il est possible soit de récupérer les boues pour les faire entrer dans la constitution d'un engrais naturel, soit de transférer ces boues vers la cuve D.

Dans cette dernière hypothèse, les boues sont transférées de la cuve **B** dans la cuve **D**, par une pompe **18**, et après passage à l'intérieur d'un deshydrateur mécanique **19** permettant de ramener la concentration en matières sèches de 116 g/l à 180 g/l, afin de réduire le volume de la cuve **D**.

La cuve **D** est, comme les autres cuves, équipée d'un compresseur non représenté au dessin, qui aspire, à intervalles réguliers, le gaz produit dans cette cuve, et l'insuffle dans des conduits **20** équipés de busettes, afin d'agiter les boues et d'assurer leur déplacement sur le fond **22** qui est incliné d'un cm/m environ de l'extrémité amont vers l'extrémité aval.

Le temps de rétention des boues dans la cuve étant de soixante jours, l'entrée et le rejet journaliers représentent 1/60e du volume des boues.

A l'entrée dans la cuve **D**, la teneur en matières sèches est de l'ordre de 180 g/l soit 71 g/l de matières organiques et 109 g/l de matières inertes. Grâce au temps de rétention très long de soixante jours la dégradation des matières organiques est de 97 à 98 %, ce qui ramène la concentration en matières organiques à environ 2 g/l, celle des matières minérales demeurant inchangée, ce qui conduit à une concentration en matières sèches de l'ordre de 111 g/l.

Dans cette cuve, la méthanisation élève le pH d'une valeur de 7,5 à environ 8, le gaz produit contenant environ 70 % de $CH_4$ et 30 % de $CO_2$.

Les boues ne contenant pratiquement plus de matières polluantes peuvent, sans inconvénient, être rejetées dans un site après avoir été extraites de la cuve **D** par une pompe **23**.

Comme il va de soi, l'invention ne se limite pas à la seule forme d'exécution de cette installation, décrite ci-dessus à titre d'exemple ; elle en embrasse, au contraire, toutes les variantes de réalisation ; c'est ainsi notamment que l'agitation dans les cuves de fermentation pourrait être réalisée par des agitateurs mécaniques, sans que l'on sorte pour autant du cadre de l'invention.

**Revendications**

· 1. - Procédé de fermentation méthanique de matières organiques, caractérisé en ce qu'il consiste à réaliser successivement l'oxygénation des boues pendant un jour, une préfermentation des boues en conditions aérobie puis anaérobie pendant un jour, une fermentation en conditions anaérobie pendant vingt jours et, éventuellement, une fermentation complète en conditions anaérobie pendant soixante jours, les boues subissant la première phase d'oxygénation étant formées par moitié par des boues préparées à partir des matières organiques à traiter et pour moitié par des boues recyclées après avoir subi la première phase de fermentation.

2.- Procédé selon la revendication 1, caractérisé en ce qu'il consiste,
- au cours de la phase d'oxygénation, à réaliser une insufflation d'air dans la cuve dans laquelle se trouvent les boues, cette insufflation étant de 60 l/heure et par $m^3$, et la teneur en matières sèches des boues étant de 180 g/l,
- au cours de la phase de préfermentation à réaliser une agitation des boues par insufflation du gaz produit par la réaction et à maintenir la température à 37° C,
- au cours de la première phase de fermentation à agiter périodiquement les boues à l'aide du gaz produit par la réaction, cette phase se déroulant dans une enceinte isolée thermiquement, et
- au cours de la seconde phase de fermentation à réaliser une agitation périodique des boues à l'aide du gaz dégagé.

3. - Procédé selon la revendication 1, caractérisé en ce qu'il consiste quotidiennement à prélever un volume de boues ayant subi la première phase de fermentation égal au volume de boues devant subir la phase d'oxygénation, à recycler la moitié de ces boues pour la phase d'oxygénation, et à diriger l'autre moitié de ces boues soit vers une installation de réalisation d'engrais, soit vers la seconde cuve de fermentation.

4. - Dispositif pour la mise en oeuvre du procédé selon l'une quelconque des revendications 1 à 3, caractérisé en ce qu'il comprend quatre cuves (A, B, C, D) dont les trois premières sont décalées verticalement les unes par rapport aux autres, la première cuve (A) qui correspond à la cuve la plus haute étant la cuve d'oxygénation, la seconde cuve (B) communiquant avec celle-ci par un conduit (4) équipé d'une vanne (5) étant la cuve de préfermentation, la troisième cuve (C) servant à la première fermentation étant reliée à la cuve précédente (B) par un conduit (10) sur lequel est montée une vanne (12), la quatrième cuve (D) étant reliée à la troisième cuve (C) par un conduit sur lequel est disposée une pompe (18), le volume de chacune des première et deuxième cuves (A, B) étant égal au vingtième du volume de la troisième cuve (C), et le volume de la quatrième cuve (D) étant inférieur au volume de la troisième cuve.

5. - Dispositif selon la revendication 4, caractérisé en ce que, sur le conduit reliant les troisième et quatrième cuves (C, D), est monté un deshydrateur (19) destiné à augmenter la concentration des boues en matrières sèches, et le volume de la quatrième cuve (D) est égal à 0,583 fois le volume de la troisième cuve (C).

6. - Dispositif selon l'une quelconque des revendications 4 et 5, caractérisé en ce qué le fond de chaque cuve (A, B, C, D) est incliné de l'extrémité amont vers l'extrémité aval de celle-ci d'une valeur de l'ordre de 1 à 2 cm/m.

7. - Dispositif selon l'une quelconque des revendications 4 à 6, caractérisé en ce que le fond de chacune des cuves (A, B, C, D) est équipé de busettes alimentées par un compresseur (3, 9, 13), les busettes (2) de la première cuve (A) étant alimentées par de l'air prélevé à l'extérieur de celles-ci, tandis que les busettes (8, 14, 20) des autres cuves (B, C, D) sont alimentées par du gaz contenu dans chacune de celles-ci.

8. - Dispositif selon l'une quelconque des revendications 4 à 7, caractérisé en ce que les cuves de préfermentation et de fermentation (B, C, D), sont équipés d'agitateurs mécaniques conformés pour assurer le déplacement des boues de l'extrémité amont vers l'extrémité aval d'une cuve.

9. - Dispositif selon l'une quelconque des revendications 4 à 8, caractérisé en ce que la seconde cuve (B) est équipée de moyens de chauffage et de régulation de la température à 37°C, tandis que la troisième cuve (C) est équipée de moyens d'isolation thermique assurant un maintien en température des boues transférées depuis la seconde cuve.

10. - Dispositif selon l'une quelconque des revendications 4 à 9, caractérisé en ce qu'à l'extrémité aval de la troisième cuve (C), c'est-à-dire de la cuve correspondant à la première phase de fermentation est connecté un conduit (17) de retour des boues vers la première cuve (A) ou cuve d'oxygénation, ce retour des boues étant réalisé à l'aide d'une pompe (16).

0290361

FIG_1

FIG_3

# FIG_2

0290361

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 440 920  (UNION CARBIDE)<br>* Page 58, revendication 1 * | 1,2 | C 02 F    3/30 |
| A | CH-A-  657 604  (UTB UMWELTTECHNIK BUCHS)<br>* Page 4, colonne de droite, ligne 27 - page 5, colonne de droite, ligne 2 * | 1 | |
| A | FR-A-2 409 305  (H. BARRETH)<br>* Page 7, revendications 1,2; page 4, ligne 2 - page 6, ligne 1 * | 1-4,9, 10 | |
| A | FR-A-2 002 488  (BIRD MACHINE CORP.)<br>* Page 14, revendication 1; figure 1 * | 5 | |
| A | WASTEWATER TREATMENT PLANT DESIGN, 1977, Water Pollution Control Federation, pages 519-527, chapitre 27, American Society of Civil Engineers, Washington, D.C., US; "Anaerobic sludge digestion"<br>* Page 519: "Detention time"; page 521: "Temperatur"; page 522: "Mixing and recirculation"; page 525: "Digester mixing"; page 526: "Gas recirculation" * | 2,7-9 | |

**DOMAINES TECHNIQUES RECHERCHES (Int. Cl.4)**

C 02 F
C 12 M

Le présent rapport a été établi pour toutes les revendications

| Lieu de la recherche | Date d'achèvement de la recherche | Examinateur |
|---|---|---|
| LA HAYE | 22-07-1988 | TEPLY J. |